# EUROPÄISCHE PATENTSCHRIFT

(11) **EP 0 631 666 B1**
(45) Veröffentlichungstag und Bekanntmachung des Hinweises auf die Patenterteilung: **18.06.1997**
(21) Anmeldenummer: 93905160.3
(22) Anmeldetag: 03.03.1993
(51) Int. Cl.: G01N 23/225

(54) **VERFAHREN ZUR BESTIMMUNG DER ELEMENTKONZENTRATION BEIM ELEKTRONENSTRAHLSCHMELZEN**
PROCESS FOR DETERMINING THE CONCENTRATION OF ELEMENTS DURING ELECTRON BEAM MELTING
PROCEDE DE DETERMINATION DE LA CONCENTRATION D'ELEMENTS LORS DE LA FUSION PAR BOMBARDEMENT ELECTRONIQUE

(30) Priorität: 19.03.1992 DE 4208955
(43) Veröffentlichungstag der Anmeldung: 04.01.1995
(73) Patentinhaber: FRAUNHOFER-GESELLSCHAFT ZUR FÖRDERUNG DER ANGEWANDTEN FORSCHUNG E.V., 80636 München (DE)
(72) Erfinder: PANZER, Siegfried, D-8021 Dresden (DE); SCHILLER, Siegfried, D-8051 Dresden (DE); SCHILLER, Nicolas, D-8351 Helmsdorf (DE)
(86) Internationale Anmeldenummer: DE9300203
(87) Internationale Veröffentlichungsnummer: WO9319360

(56) Entgegenhaltungen:
- US-A- 5 057 688

## Beschreibung

Das Verfahren dient zur in situ-Bestimmung der Elementkonzentration beim Elektronenstrahlschmelzen, um kontinuierlich während des Schmelzprozesses zu messen und damit die Möglichkeit einer sofortigen Korrektur zu schaffen. Das Verfahren wird beim Schmelzen von Legierungen angewendet.

Es sind Verfahren zur Analyse des Schmelzbades bekannt, die darauf beruhen, daß in gewissen Zeitabständen Proben aus der Schmelze entnommen werden und anschließend mit bekannten Methoden in einem Labor analysiert werden. Die Zusammensetzung der Probe wird als repräsentativ für die Zusammensetzung der Schmelze angenommen. (J.C.Borofka & Entrekin, "Recent Advances in Electron Beam Refining of Nickel-Base Superalloys", in: Electron Beam Melting and Refining, State of the Art 1991, Proc. of Conf., Englewood, Seiten 227 bis 239)
Nachteile dieses Verfahrens sind, daß es zeitlich diskontinuierlich ist und das Meßergebnis erst nach einiger Zeit zur Verfügung steht.
Zur Vermeidung dieser Nachteile sind Verfahren bekannt, die darauf beruhen, daß die beim Schmelzen mit dem Elektronenstrahl entstehende Röntgenstrahlung ausgenutzt wird. Es wird dazu die Intensität der charakteristischen Strahlung des zu bestimmenden Elements und die Intensität eines weiteren Teils aus dem Röntgenspektrum vom gleichen Ort der Schmelze gemessen. Aus diesen beiden Meßgrößen wird der Quotient gebildet, der eine Funktion der Konzentration des zu bestimmenden Elements ist. Die Messung erfolgt zweckmäßigerweise mittels eines Kristallspektrometers. Die Bezugsintensität kann auf verschiedene Weise gemessen werden. zweckmäßig ist es jedoch, die Intensität des ausgewählten Bereiches des Röntgenbremsspektrums mit dem selben Kristallspektrometer zu messen, mit dem auch die charakteristische Strahlung des zu bestimmenden Elements gemessen und durch ein spezielles Meßverfahren ermöglicht wird (DD-AP 287 576 A 5).
Des weiteren ist es bekannt, ein energiedispersives Röntzenspektrometer zur Erfassung der Röntgenstrahlen beim Elektronenstrahlschmelzen zu verwenden (Mitchell, A.; Takagi, K.: Chemical control in electron beam melting; Proc. on Electron Beam Melting and Refining - State of the Art. 1984. Reno/Nevada 1984, p. 89-99).
Bei beiden vorgenannten Verfahren unter Anwendung röntgenspektrometrischer Verfahren hat sich aber unter industriellen Bedingungen gezeigt, daß das Meßsignal durch Absorption und Emission von Röntgenstrahlen durch Metalldämpfe gestört wird. Das hat den Nachteil zur Folge, daß das Meßergebnis verfälscht ist.

Des weiteren ist das Elektronenstrahlregime häufig so gestaltet, daß das Beobachtungsfeld des Röntgenspektrometers nur kurzzeitig vom Auftreffort des Elektronenstrahls (Spot) berührt wird. Die Röntgenstrahlung, die während der Zeit gemessen wird, in der der Beobachtungsort nicht im Spot liegt, wird von gestreuten Elektronen erzeugt. Dies kann zu Meßfehlern führen.

Der Erfindung liegt die Aufgabe zugrunde, ein Verfahren zur Bestimmung der Elementkonzentration in-situ beim Schmelzen mit Elektronenstrahl zu schaffen, welches kurzzeitig ein exaktes Meßergebnis liefert. Es muß unter industriellen Bedingungen einsetzbar sein und darf gegenüber den bekannten Verfahren keinen wesentlich höheren Aufwand erfordern. Das Verfahren soll auf dem Prinzip der Auswertung der beim Schmelzen entstehenden Röntgenstrahlung basieren.

Erfindungsgemäß wird diese Aufgabe durch die Auswertung der charakteristischen Röntgenstrahlung der in der Schmelze enthaltenen Elemente durch Erfassen mit einem Röntgenspektrometer dadurch gelöst, daß in das Schmelzregime des Elektronenstrahls in bestimmten vorgegebenen Zeitintervallen in den Schmelzprozeß spezielle Meßphasen integriert sind. Nur in diesen Zeitintervallen erfolgt eine röntgenanalytische Konzentrationsbestimmung. Das bedeutet, daß während der Zeit der Meßwerterfassung das Schmelzregime derart modifiziert wird, daß keine die Messung beeinflussende Dampfwolke über der Schmelze entsteht.Dazu wird der Elektronenstrahl auf der Schmelzbadoberfläche auf einer ausgewählten kleineren Fläche so abgelenkt, daß diese vollständig beaufschlagt ist. Im Bereich dieser Fläche liegt dann das Beobachtungsfeld für die röntgenanalytische Konzentrationsbestimmung.

Der Elektronenstrahl beaufschlagt diese kleine Fläche nach einem der Schmelztechnologie angepaßten Programm und erzeugt das entsprechende Ablenkmuster. Dadurch ist gesichert, daß der Elektronenstrahl den Beobachtungsort des Spektrometers während der Meßphase berührt und optimale Meßbedingungen gegeben sind. Es ist vorteilhaft, zur Verringerung der Dampfwolke mit bekannten Mitteln die Beschleunigungsspannung und/oder Leistung und/oder Ablenkung des Elektronenstrahles abweichend von den für den Schmelzprozeß erforderlichen eingestellten Parametern zu verändern. Dadurch soll die Leistungsdichteverteilung auf dem Schmelzbad so verändert werden, daß der Dampfdruck vermindert wird. Des weiteren ist die Beschleunigungsspannung des Elektronenstrahls für die Röntgenanalytik in vielen Fällen zu hoch.

Die Steuerung der Meßphasen im gesamten Schmelzprozeß, d. h. deren Abstände und Dauer, sowie die Veränderung der Parameter des Elektronenstrahles wird in üblicher Weise mit bekannten elektronischen Mitteln durchgeführt.

Anhand eines Ausführungsbeispiels wird die Erfindung näher erläutert. In der zugehörigen Zeichnung ist schematisch eine Draufsicht auf die Schmelzbadoberfläche dargestellt.

Während des Schmelzprozesses wird die Schmelzbadoberfläche 1 im Kristallisator 2 vom Elektronenstrahl nach technologisch vorgesehenen Mustern beaufschlagt. Das können über die Oberfläche verteilt kreisförmige Ablenkmuster 3 sein.
In bestimmten zeitlichen Abständen wird über eine konstante Zeit, der sog. Meßphase, der Elektronenstrahl in die Mitte der Schmelzbadoberfläche 1 gelenkt und beaufschlagt dort in einem Raster mit verminderter Leistung ein Feld 4. Dieses Feld 4 auf der Schmelzbadoberfläche hat im Verhältnis zur gesamten Schmelzbadoberfläche sehr kleine Ausdehnungen.
In diesem Feld 4 liegt das Beobachtungsfeld 5 des an sich bekannten Röntgenspektrometers. Nach der Meßwerterfassung wird der Elektronenstrahl wieder auf der Schmelzbadoberfläche 1 programmiert abgelenkt, um den Schmelzprozeß fortzusetzen.
Die Bestimmung der Elementenkonzentration erfolgt, indem die mit dem Elektronenstrahl entstehende Röntgenstrahlung im Bereich des Beobachtungsfeldes 5 erfaßt wird. Dabei wird gleichzeitig die Intensität der charakteristischen Strahlung des zu bestimmenden Elements und die Intensität eines weiteren Teils aus dem Röntgenspektrum vom gleichen Ort des Beobachtungsfeldes 5 gemessen. Aus den beiden Meßgrößen wird der Quotient gebildet, der eine Funktion der Konzentration des zu bestimmenden Elements ist.

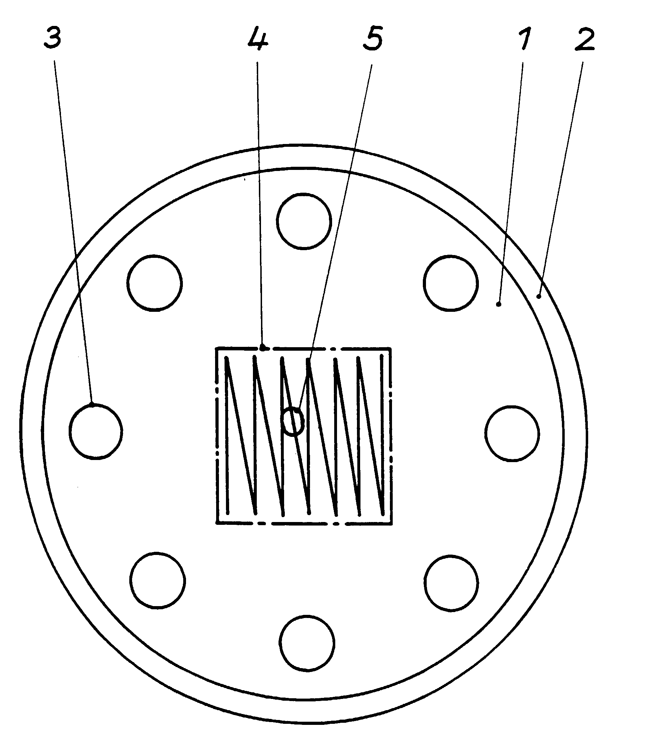

## Patentansprüche

1. Verfahren zur Bestimmung der Elementkonzentration beim Elektronenstrahlschmelzen durch Auswertung der charakteristischen Röntgenstrahlung der in der Schmelze enthaltenen Elemente, die durch ein Röntgenspektrometer erfaßt wird, **dadurch gekennzeichnet,** daß in bestimmten vorgegebenen Zeitintervallen in den Schmelzprozeß spezielle Meßphasen integriert werden, indem während der Meßphase das Schmelzregime derart modifiziert wird, daß auf der Schmelzbadoberfläche eine ausgewählte Fläche vollständig vom Elektronenstrahl beaufschlagt wird und während dieser Zeit die röntgenanalytische Konzentrationsbestimmung durchgeführt wird.

2. Verfahren nach Anspruch 1, **dadurch gekennzeichnet,** daß während der Meßphase die Beschleunigungsspannung und/oder Leistung und/oder Strahlablenkung geändert werden.

3. Verfahren nach einem der Ansprüche 1 bis 3, **dadurch gekennzeichnet,** daß die Meßphasen in den Schmelzprozeß mittels bekannter elektronischer Steuerungen integriert werden.

## Claims

1. Method of determining the concentration of elements during electron beam melting by exploitation of the characteristic X-radiation of the elements contained in the melt, said radiation being detected by an X-ray spectrometer, **characterised in that** at certain preset intervals of time, special measuring phases are integrated into the melting process, the melting process being modified during the measuring phase of the melting regime in such a way that on the surface of the melting bath a chosen area receives the full impact of the electron beam and the determination of concentration based on X-ray analysis is carried out during this time.

2. Method according to claim 1, **characterised in that** during the measuring phase the accelerating voltage and/or capacity and/or beam deflection are altered.

3. Method according to one of claims 1 to 3, **characterised in that** the measuring phases are integrated into the melting process by means of known electronic controls.

## Revendications

1. Procédé pour déterminer la concentration d'un élément à la fusion par faisceaux d'électrons, par l'exploitation du rayonnement X caractéristique des éléments contenus dans le bain et qui sont détectés par un spectromètre à rayons X,
caractérisé en ce qu'
à des intervalles de temps prédéterminés, on intègre des phases de mesure particulières au procédé de fusion, en ce qu'on modifie le régime de fusion pendant la phase de mesure et en ce qu'à la surface du bain, on sollicite complètement, avec le faisceau d'électrons, une surface sélectionnée et, pendant ce temps, on détermine la concentration par l'analyse des rayons X.

2. Procédé selon la revendication 1,
caractérisé en ce qu'
on modifie la tension d'accélération et/ou la puissance et/ou la déviation du faisceau pendant la phase de mesure.

3. Procédé selon l'une des revendications 1 et 2,
caractérisé en ce qu'
on intègre les phases de mesure dans le procédé de fusion à l'aide de commandes électroniques connues.
